# EUROPÄISCHE PATENTSCHRIFT

(11) **EP 0 457 725 B1**
(45) Veröffentlichungstag und Bekanntmachung des Hinweises auf die Patenterteilung: **08.03.1995**
(21) Anmeldenummer: 91810353.2
(22) Anmeldetag: 08.05.1991
(51) Int. Cl.: C07C 51/58

(54) **Verfahren zur Herstellung von 2,3-Dibrompropionylchlorid**
Process for the preparation of 2,3-dibromopropionyl chloride
Procédé de préparation de chlorure de 2,3-dibromopropionyl

(30) Priorität: 17.05.1990 CH 1666/90
(43) Veröffentlichungstag der Anmeldung: 21.11.1991
(73) Patentinhaber: CIBA-GEIGY AG, 4002 Basel (CH)
(72) Erfinder: Arquint, Alfons, CH-4058 Basel (CH); Leupin, Peter, Dr., CH-4051 Basel (CH)

(56) Entgegenhaltungen:
- JOURNAL FÜR PRAKTISCHE CHEMIE, Band 314, Heft 1, 1972, Leipzig, DE; F. ASINGER et al: "Synthese von (3-14C) 1-Chlor-, 1-Brom- und 1-Jod-2,3-epoxypropan", Seiten 80-86
- HELVETICA CHIMICA ACTA, Band 56, Fasc. 7, 1973, Nr. 232, Basel, CH; K. R FENACHT: "Thio- und Di-thiophosphorsäureester von der Art des GS 13005 mit einem analogen oder homologen heterocyclischen Ring", Seiten 2186-2204
- J. SOC. DYERS COLOUR., Band 104(2), 1988, R.S. DAVIDSON et al: "The efficiency of reaction between reactive fluorescent whitening agents and wool", S. 86-93

## Beschreibung

Die Erfindung betrifft ein verbessertes, einstufiges Verfahren zur Herstellung von 2,3-Dibrompropionylchlorid, das darin besteht, dass man:
a) Acrylsäure bromiert,
b) in die entstandene Schmelze Eisenpulver oder ein Eisensalz gibt,
c) die entstandene Dibrompropionsäure zum 2,3-Dibrompropionylchlorid chloriert, und
d) die flüchtigen Komponenten abtrennt.

Das nach dem erfindungsgemässen Verfahren erhaltene 2,3-Dibrompropionylchlorid wird mit hoher Ausbeute (über 97 %) und hoher Reinheit (über 98 %) erhalten.

2,3-Dibrompropionylchlorid ist u.a. als Reaktivkomponente ein wichtiges Zwischenprodukt für Reaktivfarbstoffe.

Das erfindungswesentliche Merkmal besteht darin, dass gemäss b) in die entstandene Schmelze Eisenpulver oder ein Eisensalz gegeben wird.

Im einzelnen wird die Reaktion wie folgt durchgeführt:
a) Acrylsäure (CH₂=CH-COOH) wird bromiert. Dies geschieht derart, dass man das Bromierungsmittel, vorzugsweise fl. Brom in geringem Ueberschuss (∼1 %) vorlegt und bei einer Temperatur von 20 bis 70°C Acrylsäure tropfenweise zudosiert.
b) Sobald eine Schmelze vorliegt und alle Acrylsäure zugegeben ist, wird in diese bei einer Temperatur von 20-70°C Eisenpulver oder ein Eisensalz vorteilhaft in Form einer 40 %-igen wässrigen Lösung gegeben; man verwendet vorteilhaft 0,01 bis 2,0 vorzugsweise 0,1 bis 0,3, insbesondere 0,2 Mol% Eisenpulver bzw. Eisensalz (bezogen auf die Acrylsäure). Als Eisensalze kommen vor allem in Frage: Eisen-III-chlorid, Eisen-II-chlorid, Eisen-II-iodid, Eisen-II-nitrat, Eisen-III-bromid, Eisen-II-oxid, Eisen-III-oxid, Eisen-II-hydroxid, Eisen-III-hydroxid und Eisen-II-sulfat; das bevorzugte Eisensalz ist Eisen-III-chlorid. Nach der Zugabe des Eisenpulvers bzw. des Eisensalzes erfolgt
c) die Chlorierung der Dibrompropionsäure (CH₂.Br-CH.Br-COOH) zum 2,3-Dibrompropionylchlorid (CH₂.Br-CH.Br-COCl) indem während 3 bis 10 Stunden gleichmässig bei einer Temperatur von etwa 55° bis 75°C, insbesondere 65°C unter Rühren das Chlorierungsmittel, vor allem Thionylchlorid zur Schmelze (vorteilhaft verwendet man einen Ueberschuss an Chlorierungsmittel von 1-30 %, vorzugsweise 5-25 % und insbesondere 15-22 %) zudosiert wird. Anschliessend erhält man eine Reaktionsmasse welche mehr als 95 % 2,3-Dibrompropionylchlorid enthält, und aus der man
d) die flüchtigen Komponenten vor allem HCl und SO₂ und Ueberschuss Thionylchlorid während 1-2 Stunden abtrennt, vorteilhaft durch abdestillieren z.B. bis zu einem Vakuum von 25 mbar.

Die folgenden Beispiele veranschaulichen die Erfindung ohne sie darauf zu limitieren.

Beispiel 1: 224 g Brom werden bei 15-20°C vorgelegt. Unter Rühren dosiert man innert 8 Stunden 100 g Acrylsäure gleichmässig zu, unter gleichzeitigem Temperaturanstieg und Rückfluss und rührt anschliessend noch während 30 Minuten bei 64 bis 66°C nach. Man erhält eine Schmelze der 2,3-Dibrompropionsäure. In diese Schmelze trägt man 1,1 g Eisen-III-chlorid-Lösung (40 % in Wasser) ein. Sodann werden innerhalb 5 Stunden gleichmässig bei 65°C unter Rühren 191 g Thionylchlorid (≙ 15 % Ueberschuss) zur Schmelze zudosiert. Bis zur Beendigung der Gasentwicklung rührt man noch etwa 1 Stunde bei 65°C nach. Man erhält eine rohe Reaktionsmasse mit einer Reinheit von 93-95 %, die zur Entfernung der flüchtigen Komponenten bei 65°C auf 25 mbar evakuiert und während ca. 1 1/2 h entgast wird. Man erhält so 344 g 2,3-Dibrompropionylchlorid (98 % der Theorie bezogen auf Acrylsäure) mit einer Reinheit von 98-99 %.

Beispiel 2 (Vergleichsbeispiel)
224 g Brom werden bei 15-20°C vorgelegt. Unter Rühren dosiert man innert 8 Stunden 100 g Acrylsäure gleichmässig zu unter gleichzeitigem Temperaturanstieg und Rückfluss und rührt anschliessend noch während 30 Minuten bei 64 bis 66°C nach. Man erhält eine Schmelze der 2,3-Dibrompropionsäure. Sodann werden innerhalb von 4-6 Stunden bei 65°C unter Rühren 280 g Thionylchlorid (≙75 % Uberschuss) zur Schmelze zudosiert. Bis zur Beendigung der Gasentwicklung rührt man noch 18-22 h am Rückfluss bei ansteigender Temperatur bis 85-90°C nach. Man erhält eine rohe Reaktionsmasse mit einer Reinheit von 75-80 %. Der Thionylchlorid-Ueberschuss wird bei 60-70°C und 100-200 mbar während ca. 2 Stunden abdestilliert. Zur vollständigen Entfernung der flüchtigen Komponenten wird bei 65°C auf 25 mbar evakuiert und während 1-2 Stunden entgast. Man erhält so 341 g 2,3-Dibrompropionylchlorid (95 % der Theorie bezogen auf Acrylsäure) mit einer Reinheit von 94-96 %.

| Zusammensetzung von Endprodukt im HPLC (%G) | | |
|---|---|---|
| Bezeichnung | Beispiel 1 mit Eisen-III-chlorid | Beispiel 2 ohne Eisen-III-chlorid |
| 2,3-Dibrompropionylchlorid (Hauptkomponente) | 98-99 % | 94-96 % |
| 2-Bromacrylsäurechlorid (Nebenkomponente) | < 0,2 % | 2-3 % |

## Patentansprüche

1. Verfahren zur Herstellung von 2,3-Dibrompropionylchlorid dadurch gekennzeichnet, dass man:
a) Acrylsäure bromiert,
b) in die entstandene Schmelze Eisenpulver oder ein Eisensalz gibt,
c) die entstandene Dibrompropionsäure zum 2,3-Dibrompropionylchlorid chloriert, und
d) die flüchtigen Komponenten abtrennt.

2. Verfahren gemäss Anspruch 1 dadurch gekennzeichnet, dass a) die Bromierung mit flüssigem Brom bei einer Temperatur von 20 bis 70°C erfolgt.

3. Verfahren gemäss Anspruch 1 dadurch gekennzeichnet, dass das Eisensalz gemäss b) ein Eisen-III-chlorid, Eisen-II-chlorid, Eisen-II-iodid, Eisen-II-nitrat, Eisen-III-bromid, Eisen-II-oxid, Eisen-III-oxid, Eisen-II-hydroxid, Eisen-III-hydroxid oder Eisen-II-sulfat ist.

4. Verfahren gemäss Anspruch 3 dadurch gekennzeichnet, dass das Eisensalz gemäss b) Eisen-III-chlorid ist.

5. Verfahren gemäss Anspruch 1 dadurch gekennzeichnet, dass die Chlorierung gemäss c) mit einem Ueberschuss an Chlorierungsmittel erfolgt.

6. Verfahren gemäss Anspruch 5 dadurch gekennzeichnet, dass das Chlorierungsmittel Thionylchlorid ist.

7. Verfahren gemäss Anspruch 5, dadurch gekennzeichnet, dass man
a) Acrylsäure mit flüssigem Brom bei einer Temperatur von 20 bis 70°C bromiert,
b) in die entstandene Schmelze der Dibrompropionsäure Eisen-III-chlorid in Form einer wässrigen Lösung gibt,
c) die entstandene Dibrompropionsäure mit überschüssigem (5 bis 25 %) Thionylchlorid zum 2,3-Dibrompropionylchlorid chloriert, und
d) die flüchtigen Komponenten abtrennt.

## Claims

1. A process for the preparation of 2,3-dibromopropionyl chloride, which comprises:
a) brominating acrylic acid,
b) adding iron powder or an iron salt to the resultant melt,
c) chlorinating the dibromopropionic acid obtained to 2,3-dibromopropionyl chloride, and
d) removing the volatile components.

2. A process according to claim 1, wherein a) the bromination is carried out with liquid bromine at a temperature from 20 to 70°C.

3. A process according to claim 1, wherein the iron salt in b) is an iron(III) chloride, iron(II) chloride, iron(II) iodide, iron(II) nitrate, iron(III) bromide, iron(II) oxide, iron(III) oxide, iron(II) hydroxide, iron(III) hydroxide or iron(II) sulfate.

4. A process according to claim 3, wherein the iron salt in b) is iron(III) chloride.

5. A process according to claim 1, wherein the chlorination in c) is carried out with an excess of chlorinating agent.

6. A process according to claim 5, wherein the chlorinating agent is thionyl chloride.

7. A process according to claim 5, which comprises
a) brominating acrylic acid with liquid bromine at a temperature from 20 to 70°C,
b) adding iron(III) chloride in the form of an aqueous solution to the resultant melt of the dibromopropionic acid,
c) chlorinating the dibromopropionic acid obtained with excess (5 to 25 %) thionyl chloride to 2,3-dibromopropionyl chloride, and
d) removing the volatile components.

## Revendications

1. Procédé de préparation de chlorure de 2,3-dibromopropionyle, caractérisé en ce qu'il consiste
a) à effectuer la bromation de l'acide acrylique,
b) à introduire dans la masse fondue formée, de la poudre de fer ou un sel de fer,
c) à chlorer l'acide dibromopropionique formé pour obtenir du chlorure de 2,3-dibromopropionyle et
d) à séparer les composants volatils.

2. Procédé conforme à la revendication 1, caractérisé en ce que la bromation a) est réalisée avec du brome liquide à une température de 20 à 70 °C.

3. Procédé conforme à la revendication 1, caractérisé en ce que le sel de fer de l'étape b) est le chlorure de fer(III), le chlorure de fer(II), l'iodure de fer(II), le nitrate de fer(II), le bromure de fer(III), l'oxyde de fer(II), l'oxyde de fer(III) l'hydroxyde de fer(II), l'hydroxyde de fer (III) et le sulfate de fer (II).

4. Procédé conforme à la revendication 3, caractérisé en ce que le sel de fer de l'étape b) est le chlorure de fer(III).

5. Procédé conforme à la revendication 1, caractérisé en ce que la chloration c) se fait avec un excès d'agent de chloration.

6. Procédé conforme à la revendication 5, caractérisé en ce que l'agent de chloration est le chlorure de thionyle.

7. Procédé conforme à la revendication 5, caractérisé en ce qu'il consiste
a) à réaliser la bromation de l'acide acrylique avec du brome liquide à une température comprise entre 20 et 70 °C,
b) à introduire du chlorure de fer(III) dans la masse fondue formée d'acide dibromopropionique,
c) à chlorer l'acide dibromopropionique formé avec un excès (de 5 à 25 %) de chlorure de thionyle pour obtenir du chlorure de 2,3-dibromopropionyle et
d) à séparer les composants volatils.
